# EUROPEAN PATENT APPLICATION

(11) **EP 4 606 306 A2**
(43) Date of publication of application: **27.08.2025**
(21) Application number: 25189345.9
(22) Date of filing: 15.11.2022
(51) Int. Cl.: A61B 5/367

(54) **PLANAR CATHETER WITH OVERLAPPING ELECTRODE PAIRS**

(30) Priority: 16.11.2021 US 202163279747 P; 26.10.2022 US 202217974179
(62) Divisional of application: 22207377.7
(71) Applicant: Biosense Webster (Israel) Ltd., 2066717 Yokneam (IL)
(72) Inventor: VAN NIEKERK, Pieter Emmelius, Irvine, 92618 (US); OVIEDO BUITRAGO, Andrea, Irvine, 92618 (US); SALAZAR, Henry F., Irvine, 92618 (US); BASU, Shubhayu, Irvine, 92618 (US)
(74) Representative: Carpmaels & Ransford LLP

(57) **Abstract**

An end effector having loop members with electrodes thereon and are usable with catheter-based systems to measure or provide electrical signals. The end effector can include three loop members that are non-coplanar when expanded unconstrained that become contiguous to a planar surface when the loop members are deflected against the surface, a mechanical linkage that joins the loop members at a distal vertex of the end effector. The end effector may have a lateral gap length between laterally adjacent electrodes approximately twice that of a longitudinal gap length between longitudinally adjacent electrodes. In some configurations, longitudinal electrode bipole pairs having a gap distance approximately equal to the lateral gap distance may be provided by bypassing every other electrode in the longitudinal direction.

## Description

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to provide high-density signal maps through the use of several electrodes sensing electrical activity of tissue in an area on the order of a square centimeter. For mapping within an atria or a ventricle (for example, an apex of a ventricle), it is desirable for a catheter to collect larger amounts of data signals within shorter time spans. It is also desirable for such a catheter to be adaptable to different tissue surfaces, for example, flat, curved, irregular or nonplanar surface tissue and be collapsible for atraumatic advancement and withdrawal through a patient's vasculature. It is also desirable to produce a catheter having increased electrode density and improved maneuverability within the heart of a patient. Accordingly, it may be advantageous to provide a shortened length end effector that is more maneuverable within a heart of a patient and has a higher density of electrodes. It may also be advantageous to provide a shortened length end effector that may include equidistantly spaced bipoles by bypassing every other electrode along a linear direction.

### SUMMARY

Various embodiments described herein allow high density mapping and/or ablation of tissue surface in the heart, including an atria or a ventricle, by virtue of a catheter for electrophysiology applications. The catheter may include a tubular member and an end effector. The tubular member may include a proximal portion and a distal portion defining a longitudinal axis. The tubular member may be configured to be manipulated at the proximal portion to position the distal portion into a heart of a patient. The distal portion may include a cross-section disposed about the longitudinal axis which intersects a first and second orthogonal plane that extend along the longitudinal axis. The cross section of the distal portion may include a first opening intersecting the first orthogonal plane and the second orthogonal plane and extending along the longitudinal axis, and six apertures disposed circumferentially about the first opening. Each of the six apertures may be configured to receive a spine. The end effector may be coupled to the distal portion. The end effector may include a first, second, and third loop members. Each of the loop members may include two spines and a connector that connects the two spines. The first, second, and third loop members may be configured such that each connector of the first, second, and third loop members may overlap at a distal vertex. The end effector may be configured to be delivered through vasculature in a collapsed configuration and expand in the heart to a deployed configuration such that in the deployed configuration the loop members are approximately planar.

In another embodiment, an end effector for use with a for electrophysiology applications is disclosed. The end effector may have a distal end and a proximal end. The distal end may be configured to be delivered through vasculature in a collapsed configuration and expand in the heart to a deployed configuration. The end effector may include a plurality of electrodes disposed on a plurality of equidistantly spaced spine members parallel to a longitudinal axis. The plurality of electrodes may be arranged in a grid. The plurality of equidistantly spaced spine members may form columns of the grid such that within each respective column, the plurality of electrodes may be spaced from an immediately adjacent electrode by a first gap length. The plurality of electrodes may form rows of the grid across the plurality of spine members, such that within each respective row, the plurality of electrodes may be spaced from an immediately adjacent electrode by a second gap length that measures approximately twice the first gap length.

In another embodiment, a method of use of a catheter for high density mapping and/or ablation of tissue surface in the heart is disclosed. The example method can include one or more of the following steps presented in no particular order. The method may include moving a distal portion of an elongated shaft of an end effector extending distally from the distal portion through a catheter into a heart. The elongated shaft may define a longitudinal axis and the end effector may include a plurality of electrodes disposed on a plurality of equidistantly spaced spine members parallel to the longitudinal axis. The plurality of electrodes may be arranged in a grid, and the plurality of electrodes may form rows of the grid. The method may include moving the end effector from a distal end of the catheter by manipulation of a proximal portion of the elongated shaft. The method may include contacting the plurality of electrodes carried by the end effector to heart tissue by manipulation of the proximal portion of the elongated shaft. The method may include activating one or more first electrode bipoles disposed on at least one spine member. The one or more first electrode bipoles may include two electrodes having at least a third electrode positioned between the two electrodes. The method may include receiving, while the plurality of electrodes are in contact with the heart tissue, electrical potentials from the one or more first electrode bipoles.

According to some embodiments, the method may further include deflecting the end effector at an angle relative to the longitudinal axis to contact the one or more first electrode bipoles and one or more second electrode bipoles to the heart tissue, activating one or more second electrode bipoles disposed orthogonally to the longitudinal axis along a respective row of the grid, and receiving, while the plurality of electrodes are in contact with the cardiovascular tissue, electrical potentials from the cardiovascular tissue via the one or more second electrode bipoles.

In any of the embodiments described earlier, the following features can be combined in various permutations with the embodiments as well as with each other. The two spines of each loop members may extend along the longitudinal axis, and each of the spines may include a plurality of electrodes that have at least a first electrode, a second electrode, and a third electrode disposed thereon. Each electrode may be spaced from an immediately adjacent electrode on a respective spine member by a first gap length. The first electrode of a respective spine member and the third electrode of a respective spine member may form a first bipole. Each respective electrode of a first spine may be spaced from a corresponding electrode of a second spine by a second gap length that measures approximately twice the first gap length. Each electrode of the first spine may form second bipoles with the corresponding electrode of the second spine. The two spines of the first loop member may be attached to non-adjacent apertures of the cross-section of the distal portion. The two spines of the second loop member may be attached to immediately adjacent apertures, and the two spines of the third loop members may be attached to non-adjacent apertures of the cross-section such that the first loop member and the third loop member do not intersect the second loop member. The first loop member and the third loop member may be considered outer loops and the second loop member may be considered an inner loop such that the outer loops intersect one another along the longitudinal axis and do not intersect the inner loop along the longitudinal axis. Four apertures may be disposed on one side of the second orthogonal plane with three apertures disposed on a first side of the first orthogonal plane, and three apertures disposed on a second side of the first orthogonal plane. The second loop member may be positioned on one side of the second orthogonal plane. The first loop member may lie on a first plane, the second loop member may lie on a second plane, and the third loop member may lie on a third plane, the first, second, and third planes intersecting each other. Each spine may be configured such that in the deployed configuration each spine is approximately parallel to each other such that the plurality of electrodes are positioned to define an electrode grid. Each respective electrode of the first spine may be linearly spaced along the first spine member and form respective overlapping electrode pairs. The first gap length may be between immediately adjacent electrodes measured from a center of each of the immediately adjacent electrodes. Each spine may include a plurality of spine segments each having a length equal to the first gap length less a length of a respective electrode. Each spine member may include eight electrodes. Each of the electrodes may have an electrode length measuring from approximately 100 microns to approximately 750 microns. The first gap length may include a length selected from approximately 1 millimeter, approximately 1.2 millimeters, and approximately 2.4 millimeters. The plurality of electrodes may be configured to receive electrical potentials from the heart when the end effector is in the deployed configuration. The plurality of electrodes may be configured to ablate heart tissue when the end effector is in the deployed configuration.

### BRIEF DESCRIPTION OF THE DRAWINGS

FIG. 1A is an illustration of a catheter having an end effector at a distal portion of the catheter and a proximal handle at a proximal portion of the catheter according to aspects of the present invention.
FIGS. 1B and 1C are illustrations of orthogonal side plan views of a variation of the apparatus illustrated in FIG. 1A according to aspects of the present invention.
FIGS. 2A, 2B, 2C, and 2D are illustrations depicting orientation of loop members of the end effector according to aspects of the present invention.
FIG. 3 is an illustration of a side view of the end effector according to aspects of the present invention.
FIG. 4 is an illustration of a standard length end effector as is known in the art.
FIG. 5 is an illustration of a shortened length end effector, and a corresponding electrode grid, according to aspects of the present invention.
FIGS. 6A and 6B are illustrations of views of the end effector pressed to a planar surface according to aspects of the present invention.
FIGS. 7A, 7B, and 7C are illustrations of an electrode spacing of an end effector, an end effector having a mechanical linkage connecting each loop member, and an interior lumen of a loop member, respectively, according to aspects of the present invention.
FIG. 8 is an illustration of a cardiovascular treatment utilizing the apparatus according to aspects of the present invention.
FIG. 9 is a flowchart of an exemplary method of treating a patient utilizing the apparatus according to aspects of the present invention.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the pertinent art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

As used herein, the terms "patient," "host," "user," and "subject" refer to any human or animal subject and are not intended to limit the systems or methods to human use, although use of the subject invention in a human patient represents a preferred embodiment.

As used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

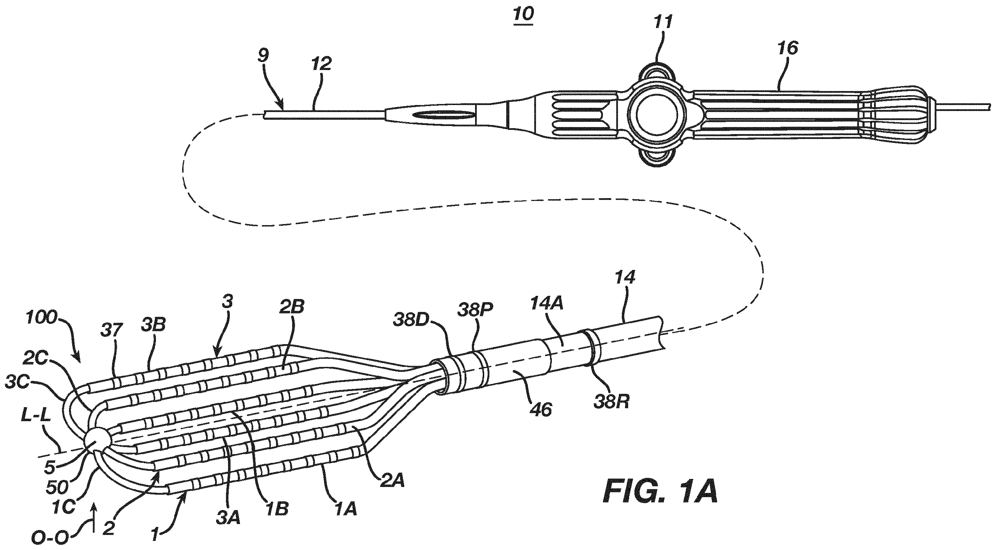

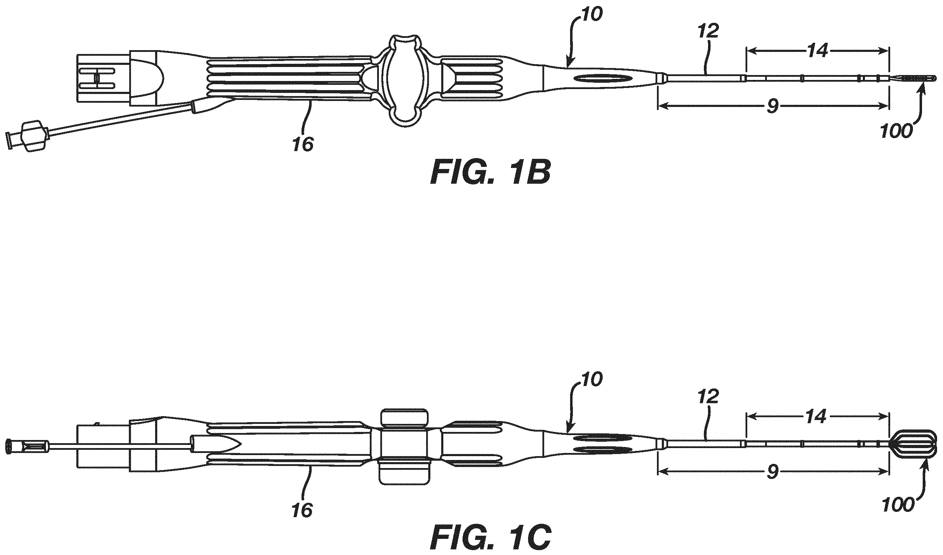

FIG. 1A illustrates an example apparatus 10 having an elongated shaft 9, a distal electrode assembly or end effector 100, and a deflection control handle 16. FIGS. 1B and 1C and illustrations of orthogonal side plan views of a variation of the apparatus 10 illustrated in FIG. 1A. The apparatus 10 can have several design variations while including novel aspects illustrated herein. The apparatus 10 is presented for illustration purposes only and is not intended to be limiting.

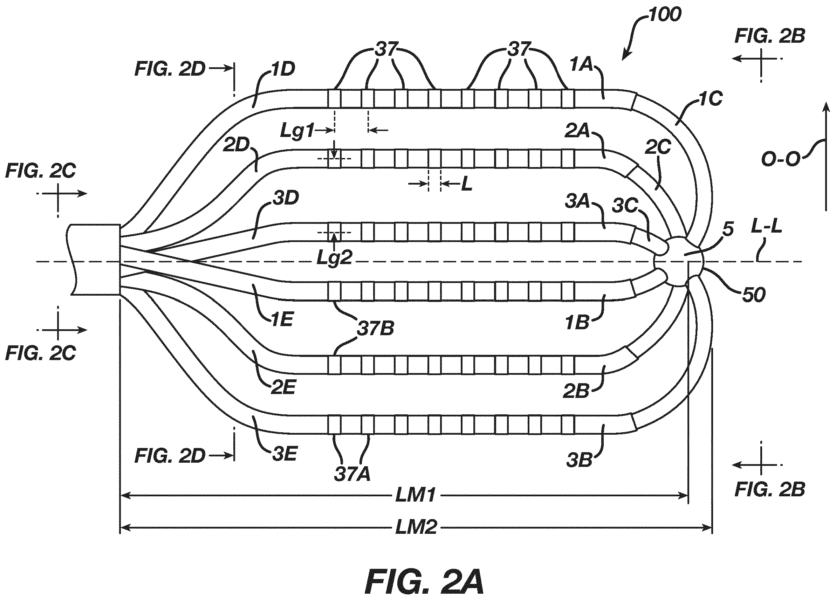

The elongated shaft 9 has a proximal portion 12 in the shape of an elongated catheter body, an intermediate deflection section 14, and distal portion 14A. The deflection control handle 16 is attached to the proximal end of the catheter body 12. The distal portion 14A of the shaft is coupled to the end effector 100 via a connector tubing 46. The elongated shaft 9 forms a tubular catheter body sized and otherwise configured to traverse vasculature. The end effector 100 has a plurality of loop members 1, 2, 3 that overlap at a common distal vertex 5 and are joined at the distal vertex 5 with a mechanical linkage 50, as shown in FIG. 2A. Catheter body 12 can be of any suitable construction and made of any suitable material. In some embodiments, the catheter body may include an outer wall made of polyurethane or PEBAX. The outer wall may include an imbedded braided mesh of stainless steel or the like to increase torsional stiffness of the catheter body 12 such that, when the control handle 16 is rotated, the intermediate section 14 of the catheter 10 may rotate in a corresponding manner. The outer diameter of the catheter body is preferably no more than about 8 French, and more preferably about 7 French. The thickness of the outer wall may be thin enough such that a central lumen within catheter body 12 may house any desired wires, cables, and/or tubes.

When the device is unconstrained and aligned, the proximal portion 12, intermediate section 14, distal portion 14A, and end effector 100 are generally aligned along a longitudinal axis L-L, as shown in FIG. 1A. The intermediate section 14 can be configured to bend to deflect the distal portion 14A and end effector 100 from the longitudinal axis L-L.

The useful length of the elongated shaft 9, i.e., that portion of the apparatus 10 that can be inserted into the body excluding the end effector, can vary as desired. Preferably the useful length ranges from about 110 cm to about 120 cm. The length of the intermediate section 14 is a relatively smaller portion of the useful length, and preferably ranges from about 3.5 cm to about 10 cm, more preferably from about 5 cm to about 6.5 cm.

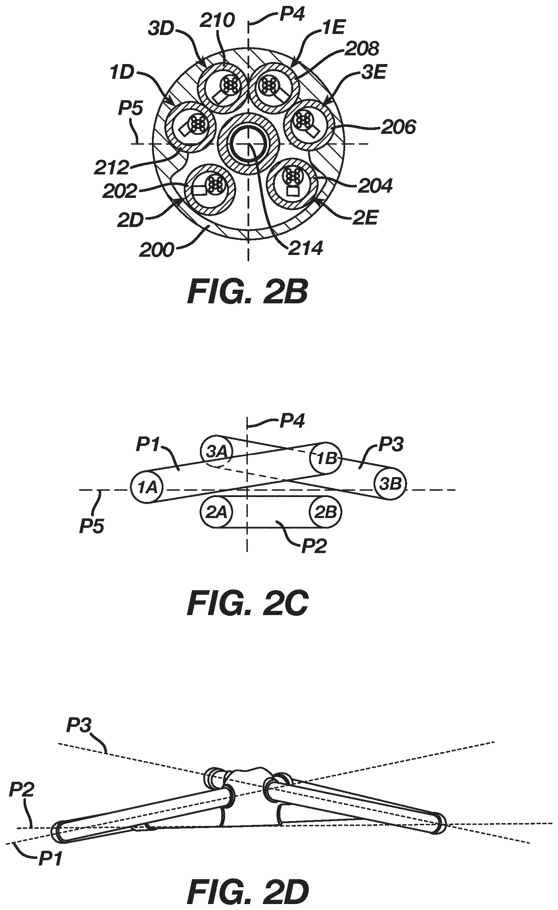

Catheter body proximal portion 12 can be attached to the intermediate section 14 as shown and described in FIGS. 2A and 2B of US Patent No. 9,820,664, which is incorporated by reference herein with a copy in the Appendix of priority application U.S. 63/279,747. If desired, a spacer (not shown) can be located within the catheter body 12 between the distal end of the stiffening tube (if provided) and the proximal end of the intermediate section 14. The spacer can provide a transition in flexibility at the junction of the catheter body 12 and intermediate section 14, which can allow this junction to bend smoothly without folding or kinking. A catheter having such a spacer is described in U.S. Pat. No. 5,964,757, the disclosure of which is incorporated herein by reference with a copy provided in the Appendix of priority application U.S. 63/279,747.

As shown in FIG. 1A, the distal portion 14A of the shaft 9 can be substantially contiguous with the intermediate section 14 such that the intermediate section includes the distal portion 14A; the distal portion being distinguished from the intermediate section 14 by the positioning of one or more (optional) ring electrodes 38R. As referred to herein, the distal portion 14A of the shaft 9 can therefore correspond to a distal portion of the intermediate section 14.

As shown in FIG. 1A, the distal portion 14A of the shaft 9 is coupled to the end effector 100 with a connector tubing 46. The connector tubing 46 includes an insert for connection of loop members 1, 2, 3 to provide electrical connection through the intermediate portion 14 of the catheter body. The connector tubing 46 can be affixed to the distal portion 14A of the catheter by glue or the like. The connector tubing 46 can be shaped to house various components such as an electromagnetic position sensor, a puller wire anchor, ring electrodes 38D, 38P, etc. The connector tubing 46 may include an internal lumen configured to house various components, including lead wires configured to connect to ring electrodes 38R, 38D, and 38P. Connector tubing 46 may additionally house lead wires configured to connect to electrodes 37 carried on the spines 1A, 1B, 2A, 2B, 3A, 3B. The internal lumen of connector tubing 46 may additionally house a cable for an electromagnetic position sensor. Electromagnetic location sensing technique is described in US Patent Nos. 5,391,199; 5,443,489; 5,558,091; 6,172,499; 6,590,963; and 6,788,967 which are incorporated by reference herein and attached to the Appendix of priority application U.S. 63/279,747. The magnetic position sensor can be utilized with impedance sensing electrode 38R in a hybrid magnetic and impedance position sensing technique known as ACL described in US Patent Nos. 7,536,218; 7,756,567; 7,848,787; 7,869,865; and 8,456,182, which are incorporated by reference herein and attached to the Appendix of priority application U.S. 63/279,747.

The end effector 100 can be collapsed (compressed toward the longitudinal axis L-L) to fit within a guiding sheath or catheter (not illustrated). The shaft 9 can be pushed distally to move the end effector 100 distally through the guiding sheath. The end effector 100 can be moved to exit a distal end of the guiding sheath via manipulation of the shaft 9 and/or control handle 16. An example of a suitable guiding sheath for this purpose is the Preface Braided Guiding Sheath, commercially available from Biosense Webster, Inc. (Irvine, California, USA).

The end effector 100 has first, second and third loop members 1, 2, and 3. Each loop member 1, 2, 3 has two spines 1A, 1B, 2A, 2B, 3A, 3B and a connector 1C, 2C, 3C that connects the two spines of the respective loop member 1, 2, 3. Spines 1A, 1B of a first loop member 1 are connected by a first connector 1C; spines 2A, 2B of a second loop member 2 are connected by a second connector 2C; and spines 3A, 3B of a third loop member 3 are connected by a third connector 3C.

For each loop member 1, 2, 3 the spines 1A, 1B, 2A, 2B, 3A, 3B in the respective pair of spines can be substantially parallel to each other along a majority of their respective lengths when the end effector 100 is expanded in an unconstrained configuration as illustrated in FIG. 1. Preferably, all spines in the end effector are parallel to each other along the majority of their respective lengths when the end effector 100 is in the unconstrained configuration. Even when all spines are parallel, the spines are not necessarily all coplanar as described in greater detail elsewhere herein, for instance in relation to FIGS. 2A through 2D.

Each spine 1A, 1B, 2A, 2B, 3A or 3B can have a length ranging between about 5 and 50 mm, preferably about 10 and 35 mm, and more preferably about 28 mm. The parallel portions of each spine 1A, 1B, 2A, 2B, 3A, 3B can be spaced apart from each other by a distance ranging between about 1 mm and 20 mm, preferably between 2 mm and 10 mm, and more preferably about 4 mm. Each spine 1A, 1A, 1B, 2A, 2B, 3A, 3B preferably carries at least eight electrodes per spine member. The end effector preferably includes six spines as illustrated. With eight electrodes on six spines, the end effector 100 includes forty-eight electrodes.

A distal electrode 38D and a proximal electrode 38P are positioned near the distal portion 14A of the shaft 9. The electrodes 38D and 38P can be configured to cooperate (e.g. by masking of a portion of one electrode and masking a different portion on the other electrode) to define a referential electrode (an electrode that does not contact tissues). One or more impedance sensing electrodes 38R can be configured to allow for location sensing via impedance location sensing technique, as described in US Patent Nos. 5,944,022; 5,983,126; and 6,445,864, which are incorporated by reference and attached to the Appendix of priority application U.S. 63/279,747.

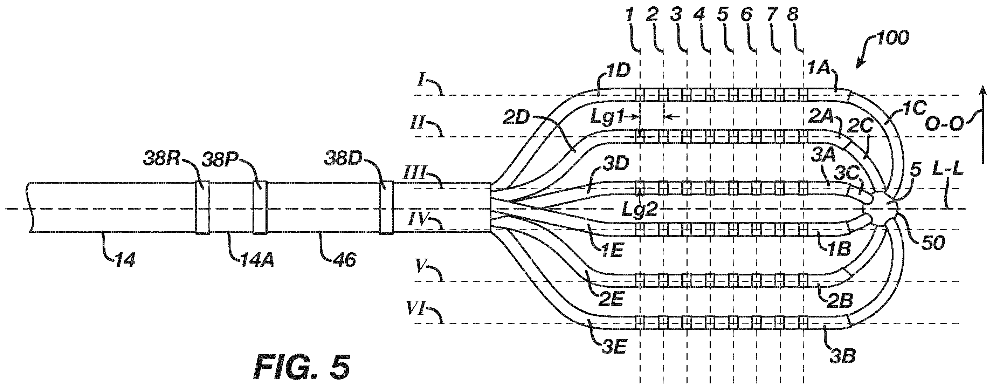

FIGS. 2A through 2D are illustrations depicting orientation of loop members of the shortened end effector. FIGS. 2B and 2C are cross sectional views of the end effector 100 and as indicated in FIG. 2A. FIG. 2D is a view of the end effector 100 looking proximally from a distal end of the end effector 100 as indicated in FIG. 2A. As shown in FIG. 2A, each of the loop members 1, 2, 3, respectively include proximal end segments 1D, 2D, 3D, 1E, 2E, 3E. affixed to the distal portion 14A of the elongated shaft 9 of the apparatus 10. The three loop members 1, 2, 3, overlap at a common distal vertex 5 along the longitudinal axis L-L. As shown, each loop member 1, 2, 3 has two spines 1A, 1B, 2A, 2B, 3A, 3B and a connector 1C, 2C, 3C that connects the two spines of the respective loop member 1, 2, 3. Each spine segment 1A, 1B, 2A, 2B, 3A, and 3B have disposed thereon one or more electrodes 37. As shown, each spine 1A, 1B, 2A, 2B, 3A, 3B carries at least eight electrodes per spine. The end effector preferably includes 6 spines as illustrated. Each electrode 37 on a respective spine may be separated from an adjacent spine by a distance of Lg1, as measured from a center of a first electrode 37 to a center of an immediately adjacent second electrode 37 (see FIG. 5). According to some embodiments, the distance between each immediately adjacent electrode may be between approximately 1 mm and approximately 2.4 mm, and preferably approximately 1.2 mm. Each electrode 37 may have an electrode width L, which may be between approximately 100 microns to approximately 750 microns, and preferably approximately 500 microns. Loop 2 of end effector 100 may have a length LM1 as measured from the base of end effector 100 to the distal vertex 5. Loop 1 and loop 3 may have a length LM2 as measured from the base of end effector 100 to the distal vertex 5. As illustrated, length LM2 may be greater than length LM1. According to some embodiments, LM1 may be approximately 17 mm to approximately 24 mm, and LM2 may be approximately 18 mm to approximately 25 mm. Each spine 1A, 2A, 3A, 1B, 2B, 3B, may form rows of a rectangular electrode grid when the electrodes 37 are in contact with the cardiovascular tissue. Along any given spine 1A, 2A, 3A, 1B, 2B, 3B, electrodes 37 may form longitudinal electrode pairs 37A between immediately adjacent electrodes 37. Between any immediately adjacent spines (e.g., 1A & 2A, 2A & 3A, 3A & 1B, 1B & 2B, and 2B & 3B) electrode 37 on a first spine and a corresponding electrode 37 on a second spine may form lateral electrode pairs 37B. As discussed above, longitudinal electrode pairs 37A may consist of immediately adjacent electrodes 37 separated by distance Lg1. Lateral electrode pairs 37B may consist of laterally adjacent electrodes 37 separated by a distance Lg2. According to some embodiments, end effector may be characterized by the distance Lg2 between lateral electrode pairs 37B that is approximately twice that of distance Lg1 between longitudinal electrode pairs 37A.

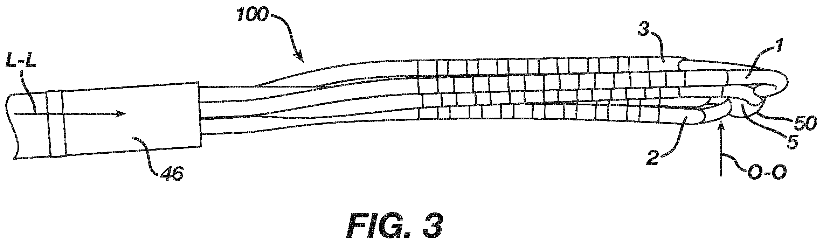

FIG. 2B illustrates a cross-sectional view through the connector tubing 46. The connector 46 includes a tubular insert 200 that has its center coinciding with the longitudinal axis L-L. Orthogonal planes P4 and P5 are in alignment with the longitudinal axis to define four quadrants in the insert 200. A parallel reference plane P5 is approximately parallel to the end effector 100 illustrated in FIG. 3, below. An orthogonal reference plane P4 is approximately orthogonal to the parallel reference plane P5 and approximately parallel to the orthogonal axis O-O. Apertures 202, 204, 206, 208, 210, 212 of the insert 200 are sized, positioned, and otherwise configured for insertion of respective end segments 2D, 2E, 3E, 1E, 3D, 1D. Apertures 202, 204, 206, 208, 210, 212 may be disposed circumferentially about a center opening 214. Center opening 214 is disposed at the intersection of orthogonal plane P4 and orthogonal plane P5 for insertion of puller wires, electrical wires, and/or as any other components to and from the end effector 100. According to some embodiments, center opening 214 may house an irrigation port for providing irrigation to the vasculature or heart of a patient. Components which traverse the apertures 202, 204, 206, 208, 210, 212 and central opening 214 are not illustrated in FIG. 2B for the purposes of illustration.

With this arrangement of apertures 202, 204, 206, 208, 210 and 212, loop members 1, 2 and 3 are arrayed in a non-coplanar unconstrained arrangement, shown in the sectional view of FIG. 2C (as viewed from the proximal end), whereby loop 3 defines a plane P3 (demarcated by spines 3A and 3B with connector 3C) that intersects orthogonal plane P4 with loop 1 having a plane P1 (demarcated by spines 1A and 1B with connector 1C) that intersects both orthogonal planes P4 and P5 and loop 2 having a plane P2 (demarcated by spines 2A and 2B and connector 2C) that intersects orthogonal plane P4 and is substantially parallel to orthogonal plane P5. FIG. 2D is a view of the distal end of the end effector looking proximally. In particular, loop 1 (defined by spines 1A, 1B and connector 1C) is arrayed to define plane P1 that is contiguous to or extend through spines 1A, 1B and loop 1 whereas spines 2A, 2B and connector 2C of loop 2 are arrayed to define a plane P2 that intersects with plane P1. Spines 3A, 3B and connector 3C of loop 3 are arrayed to define a plane P3 that intersects with both planes P1 and P2. The planes P1, P2, and P3 defined by the respective loops 1, 2 and 3 are configured so that the loops 1, 2 and 3 are not contiguous to or arrayed such that a common plane passes through the loops. Thus, planes P1, P2 and P3 are non-parallel and intersect each other. It is noted that the longitudinal axis L-L may be contiguous to the second plane P2. In alternative embodiment, longitudinal axis L-L may be disposed in between a region bounded by planes P1, P2 and P3.

FIGS. 2A through 2D are one example of non-coplanar arrangement of loop members 1, 2, 3 in an end effector. There are numerous possible arrangements non-coplanar arrangements of loop members which can result in an end effector having the general appearance of the illustrated end effector 100.

FIG. 3 is an illustration of a side view of the end effector 100. The three loop members 1, 2, 3 overlap at a common distal vertex 5 along the longitudinal axis L-L. The end effector 100 is in an unconstrained configuration as illustrated in FIG. 3. When the end effector is unconstrained, the loop members 1, 2, 3 are not coplanar with each other. FIG. 3 also illustrates an orthogonal axis O-O orthogonal to the longitudinal axis L-L and approximately orthogonal to the end effector 100.

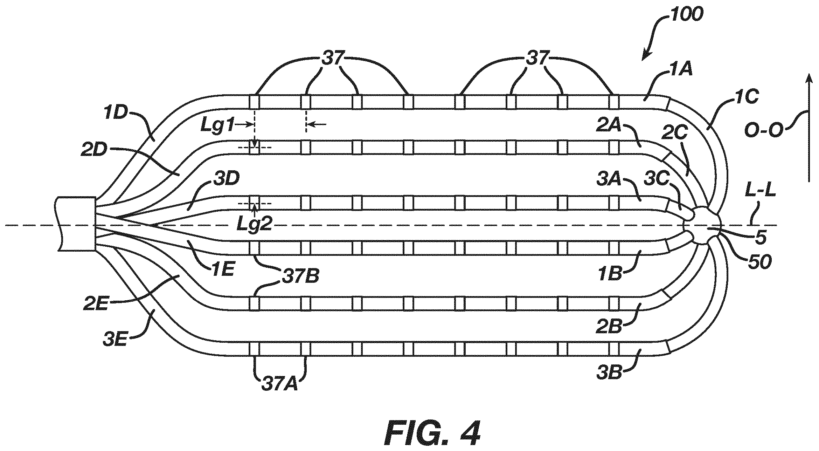

FIG. 4 is an illustration of a standard length end effector according to aspects of the present invention. As compared to the shortened end effector illustrated in FIG. 2, the standard length end effector has a different geometry as defined by the spacing between adjacent spines of the end effector 100. For example, longitudinal electrode pairs 37A along any given spine 1A, 2A, 3A, 1B, 2B, 3B, have a gap length Lg1, while lateral electrode pairs 37B between two adjacent spines have a gap length Lg2. While the shortened end effector 100 may exhibit a lateral gap length Lg2 that is approximately twice the longitudinal gap length Lg1, the standard length end effector may exhibit a lateral gap length Lg2 approximately equal to the longitudinal gap length Lg1. Advantages of the shortened end effector design may include increased electrode density, improved maneuverability of the end effector within the heart and vasculature, improved conformance to the tissue while the end effector 100 is in the deployed configuration, and improved usability within patients that have more compact cardiac anatomies.

FIG. 5 is an illustration of the shortened length end effector 100. As shown in FIG. 5, electrodes 37 disposed on spines 1A, 2A, 3A, 1B, 2B, and 3B may define an electrode grid. For example, rows I, II, III, IV, V, and VI define the rows of the electrode grid, and numbers 1, 2, 3, 4, 5, 6, 7, 8 define the columns of the electrode grid. As shown, the electrode grid includes 48 electrodes 37. The electrode grid may be substantially rectangular or square in shape, thereby allowing electrical signals propagating in orthogonal directions (e.g., a first electrical signal propagating along one of rows I-VI and a second electrical signal propagating along one of columns 1-8) to be measured with a high resolution due to the (rectangular or square) grid that end effector 100 provides. As discussed with respect to FIG. 2, shortened end effector may exhibit a lateral gap distance Lg2 that is approximately twice the longitudinal distance Lg1. Nevertheless, shortened end effector may provide equidistant electrode spacing by bypassing every other electrode in the longitudinal direction to provide for equidistantly spaced electrode bipoles in both the longitudinal (e.g., along rows I-VI) and the lateral (e.g., along columns 1-8) directions. For example, along any given row I, II, III, IV, V, and VI there exist numerous longitudinal electrode bipole pairs 37A that have a gap distance equivalent to the lateral gap distance Lg2 (e.g., pair 1-3, pair 2-4, pair 3-5, pair 4-6, pair 5-7, and/or pair 6-8 along any given row I-VI). By providing lateral electrode bipole pairs as described above, shortened end effector 100 exhibits equidistantly spaced electrode bipole pairs in both the lateral and longitudinal directions while providing a more compact and easier to maneuver end effector for imaging the heart and surrounding vasculature. Closely-spaced electrode bipoles 37 allow for more accurate detection of near field pulmonary vein potential versus far field atrial signals, which is important when treating atrial fibrillation. More specifically, near pulmonary vein potentials are very small signals whereas atrial signals are typically far larger. Accordingly, a physician may encounter difficulty in determining whether a measured potential is due to a small, close potential (e.g., from the pulmonary vein) or a larger, albeit more distant potential (e.g., from the atria). The closely spaced electrode bipoles enabled by the shortened end effector 100 allow an operating physician to more accurately determine whether he is measuring a close signal or a far signal. Accordingly, by providing closely-spaced electrodes, the physician is able to target exactly the locations of myocardial tissue that have pulmonary vein potentials and therefore allows the physician to delivery therapy to the appropriate tissue. Additionally, the closely-spaced electrode electrodes allow the physician to determine the anatomical location of the ostium/ostia based on the measured electrical signal.

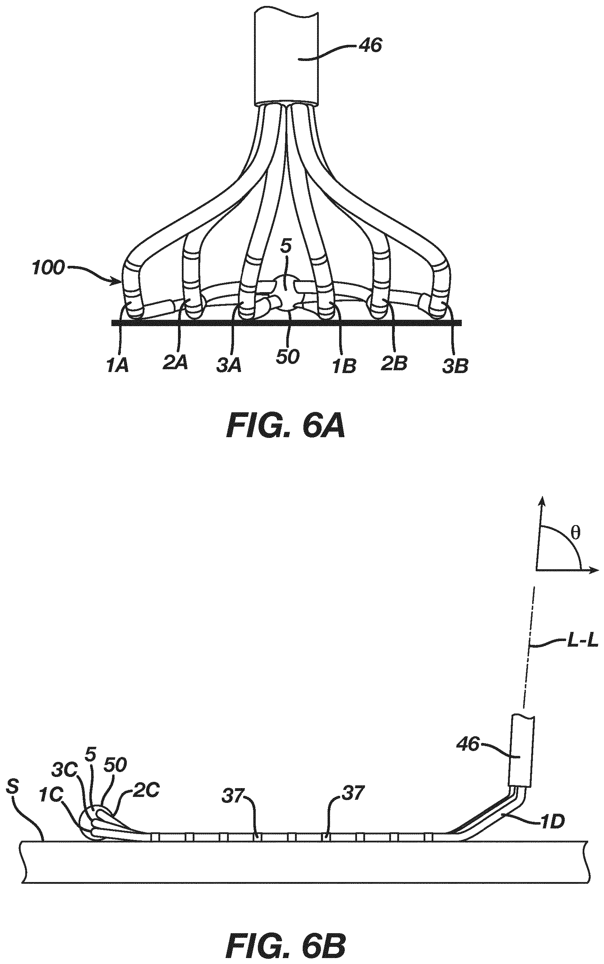

FIGS. 6A and 6B are illustrations of views of the end effector pressed to a planar surface. In the illustrated example, the loop members 1, 2, 3 can be pressed to a planar surface S via manipulation of the shaft 9 of the device. More specifically, when the end effector 100 is positioned within a patient, manipulation of the catheter body 12 and the control handle 16 can be used to position the end effector 100 against a surface within a wall of an internal cavity of the patient such internal walls of the heart and/or blood vessels. When the end effector 100 is positioned against the planar surface S, a majority of each length of each spine 1A, 2A, 3A, 1B, 2B, 3B can become contiguous and aligned to the planar surface. Further, when the end effector 100 is positioned against the planar surface S, a majority of each length of each spine 1A, 2A, 3A, 1B, 2B, 3B can become aligned with a majority of each length of the other spines. The surface S need not necessarily be planar in order for the spines 1A, 2A, 3A, 1B, 2B, 3B to become contiguous and aligned to the surface. The end effector 100 may be able to conform to a curved surface, for instance.

As illustrated in FIG. 6B, when the majority of each spine 1A, 2A, 3A, 1B, 2B, 3B is pressed to the surface S, the connecting segments 1C, 2C, 3C can be stacked on top of the surface S at the distal vertex 5 at the linkage 50. A first connecting segment 1C nearest to the surface S can be separated from the surface S by the linkage 50. A second connecting segment 3C stacked onto the first connecting segment 1C can be separated from the surface S by the linkage 50 and the first connecting segment 1C. A third connecting segment 2C can be separated from the surface S by the linkage 50 and the first and second connecting segments 1C, 3C. Therefore, at least a portion of each connecting segment 1C, 2C, 3C can be separated from the planar surface S when the majority of each spine 1A, 2A, 3A, 1B, 2B, 3B is pressed to the planar surface. Alternatively, the linkage 50 can be inset into the first connecting segment 1C such that the first connecting segment is substantially contiguous to the planar surface S. In that case, only the second and third connecting segments 3C, 2C are separated from the planar surface S at the distal vertex 5.

Proximal segments 1D, 2D, 3D, 1E, 2E, 3E of the loop members 1, 2, 3 can be bent such that at least a portion of each of the proximal segments curves away from the surface S.

When the majority of each spine 1A, 2A, 3A, 1B, 2B, 3B is pressed to the surface S, at least some of the electrodes 37 on each spine can be in contact with the surface S. In some examples, every electrode 37 on each spine can be in contact with the surface S.

When the majority of each spine 1A, 2A, 3A, 1B, 2B, 3B is pressed to the surface S, the majority of each respective length of each loop member can become contiguous to the surface S, where the respective length of each loop member includes the length of the respective loop member's spines 1A, 2A, 3A, 1B, 2B, 3B, connectors 1C, 2C, 3C, and proximal segments 1D, 2D, 3D, 1E, 2E, 3E (distal to the connector tubing 46).

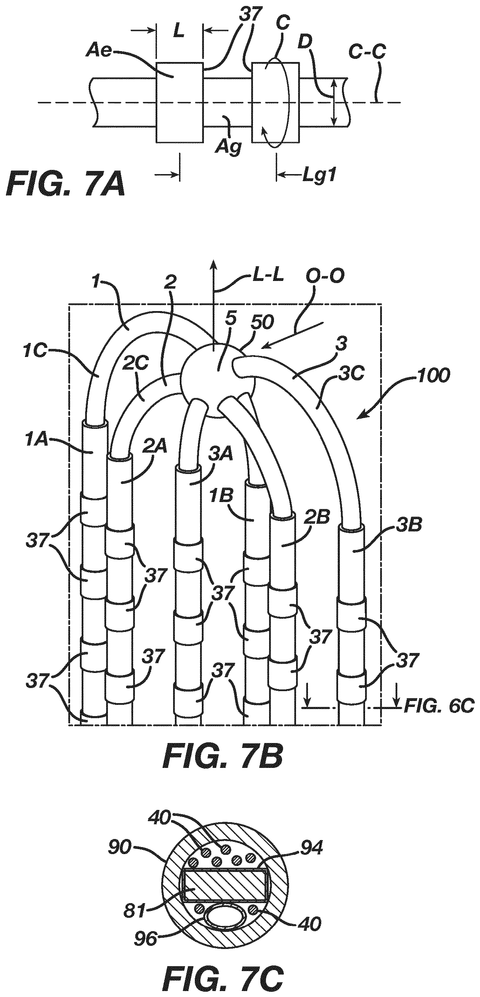

FIG. 7A is an illustration depicting electrode spacing and dimensions on the shortened end effector. The electrodes 37 can include one or more pairs of closely-spaced bipolar electrodes 37 which are configured to pick up electrocardiogram signals from tissue. In the present example, the electrodes 37 of an adjacent longitudinal pair has a separation space gap distance Lg1 therebetween of approximately 1 mm to 200 microns and preferably no greater than about 200 microns. Each electrode 37 has an electrode area Ae and electrode length L. The electrode length can be from about 2 mm to about 0.5 mm. Each electrode 37 preferably has a length of 1 mm to 0.5 mm. The electrodes 37 as illustrated are cylindrical such that the electrode area is calculated as a produce of the circumference C and length L of the electrode. The spine has a diameter D. As described with reference to FIG. 2A and FIG. 5, according to some embodiments, laterally spaced electrode pairs 37B may have a gap distance Lg2. In some embodiments, the lateral gap distance Lg2 may be approximately twice the longitudinal gap distance Lg1.

Additionally, or alternatively, the electrodes 37 need not completely circumscribe the respective loop 1, 2, 3; in which case the electrodes 37 can have a rectangular shape that is rectilinear or arced having a width W such that the electrode area Ae is a produce of the electrode length L and width W, the width being the arc length when the rectangular shape is arced. In examples where the electrode pair configurations are in shapes other than rectilinear, rectangular, or cylindrical, a conversion factor CF may be used to determine the appropriate gap distance between the electrodes based on the known area of either one of the pair of electrodes. The conversion factor CF may range from about 2 to 0.1 in the inverse of the same root dimensional unit as the planar area of an electrode. In one example, where the planar area of one electrode is about 0.08 squared-mm, the smallest gap distance Lg1 along the longitudinal axis extending through both electrodes can be determined by applying the conversion factor CF (in the inverse of the same root dimensional unit of the area or mm) to arrive at a gap distance Lg1 of about 100 microns. In another example where the area of one electrode is 0.24 squared-mm, the conversion factor CF (in the inverse of the same root dimensional unit or mm⁻¹) can be 1.25mm⁻¹ or less, giving the range of the smallest gap distance Lg1 from about 300 microns to about 24 microns. Regardless of the shape of the electrodes, a preferred conversion factor CF is about 0.8 (in the inverse of the same root dimensional unit for the electrode area).

FIG. 7B is another illustration of the shortened end effector 100 including a mechanical linkage 50. At least one pair of closely-spaced bipolar microelectrodes 37 is provided on each spine 1A, 2A, 3A, 1B, 2B, 3B in the present example. More particularly, each spine 1A, 2A, 3A, 1B, 2B, 3B carries four pairs of adjacent bipolar microelectrodes 37 corresponding to eight microelectrodes 37 per spine. This number may be varied as desired. As discussed with respect to FIG. 5, in some embodiments, the end effector 100 may provide electrode bipoles that bypass every other electrode in the longitudinal direction to provide for equidistantly spaced electrode bipoles in both the longitudinal (e.g., along rows I-VI of FIG. 5) and the lateral (e.g., along columns 1-8 of FIG. 5) directions. FIG. 5 also illustrates a mechanical linkage 50 coupling connectors 1C, 2C, 3C together in a single connection point. The mechanical linkage 50 functions to maintain a spatially fixed arrangement between the loops 1, 2, 3 at the common distal vertex 5.

FIG. 7C is an illustrations of an interior loop member lumen. As shown in FIG. 7C, interior loop member lumen may include an inner housing 94 and outer housing 90 surrounding a support frame 81. The illustrated interior loop member lumen can be used in loop members 1, 2, 3 illustrated and otherwise disclosed herein according to the teachings herein. The outer housing 90 can include polymeric tube (e.g. thermoplastic polyurethane) having a single lumen sized to house the support frame 81 and wires 40 that provide electrical connections to the end effector electrodes 37. The lumen of the outer housing 90 can also be sized to house an irrigation tube 96 having an irrigation lumen therethrough. In this configuration, the support frame is surrounded by a sleeve to isolate its edges from damaging the conductors. The inner housing 94 can be shaped, sized, and otherwise configured to closely surround the support frame 81. The inner housing 94 can include a polymeric material, for example a shrink sleeve applied during a reflow process. Because neither the wires 40 nor the irrigation tube 96 need extend beyond the spines of the loop member, the outer housing 90 need not cover the connector segment of the loop member. Within the outer housing 90, the inner housing 94 can be bonded to the outer housing 90. The bond between the inner housing 94 and outer housing 90 can inhibit fluid leakage. The bond between the inner housing 94 and outer housing 90 can promote conformity of the shape of the outer housing 90 to that of the support frame 81 inhibiting shifting of the support frame 81 within the outer housing 90 when the end effector 100 is deformed from its unconstrained configuration.

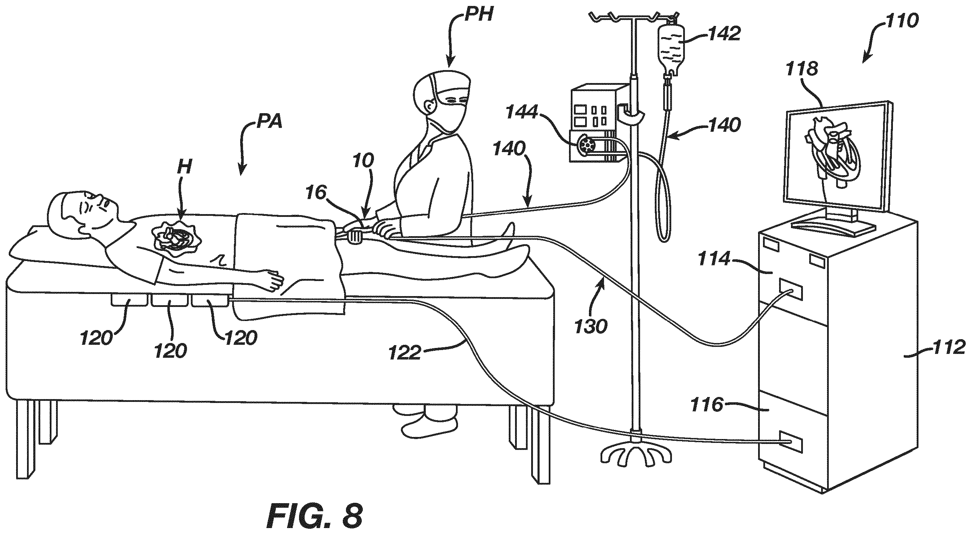

FIG. 8 is an illustration of an exemplary medical procedure and associated components of a cardiac electrophysiology (EP) mapping catheter system that may utilize the apparatus 10 (also referred to as a catheter assembly). A physician PH is illustrated grasping the handle 16 of the apparatus, with the end effector 100 (not shown in FIG. 8) disposed in a patient PA to perform EP mapping in or near the heart H of the patient PA. The apparatus 10 is coupled with a guidance and drive system 110 via a cable 130. The apparatus 10 can optionally be coupled with a fluid source 142 via a fluid conduit 140. A set of field generators 120 are positioned underneath the patient PA and are coupled with guidance and drive system 110 via another cable 122. Field generators 120 are also optional.

The guidance and drive system 110 can include a console 112 and a display 118. The console 112 can include a first driver module 114 and a second driver module 116. The first driver module 114 can be coupled with the apparatus via a cable 130. In some variations, the first driver module 114 is operable to receive EP mapping signals obtained via electrodes 37 of end effector 100. The console 112 can include a processor (not shown) that processes such EP mapping signals and thereby provides EP mapping. In addition, or in the alternative, the first driver module 114 may be operable to provide RF power to the electrodes 37 of end effector 100 to thereby ablate tissue. In some versions, the first driver module 114 is also operable to receive position indicative signals from a position sensor in end effector 100. In such versions, the processor of console 112 is also operable to process the position indicative signals from the position sensor to thereby determine the position of the end effector 100 within the patient PA.

The guidance and drive system 110 can further include non-transitory computer readable medium with instructions thereon to cause the drive system 110 to perform functionality described herein and/or as are known related to use of similar equipment. In some examples, the non-transitory computer readable memory can be in communication with the first driver module 114 (e.g. by virtue of being in communication with a processor of the first driver module 114 and/or the processor of the console 112). The non-transitory computer readable medium can include instructions thereon that when executed by the first driver module 114 cause the first driver module 114 to receive EP mapping signals from the spine electrodes 37 and a reference signal from one of distal electrode 38D, proximal electrode 38P, or a combinations of both.

The second driver module 116 is coupled with field generators 120 via a cable 122. The second driver module 116 is operable to activate field generators 120 to generate an alternating magnetic field around the heart H of the patient PA. For instance, the field generators 120 may include coils that generate alternating magnetic fields in a predetermined working volume that contains the heart H.

Some versions of the apparatus 10 include a position sense near or within the end effector 100 that is operable to generate signals that are indicative of the position and orientation of end effector 100 within the patient PA. Each position sensor may include a wire coil or a plurality of wire coils (e.g., three orthogonal coils) that are configured to generate electrical signals in response to the presence of an alternating electromagnetic field generated by field generators 120. Other components and techniques that may be used to generate real-time position data associated with end effector 100 may include wireless triangulation, acoustic tracking, optical tracking, inertial tracking, and the like. By way of example only, position sensing may be provided in accordance with at least some of the teachings of U.S. Pat. No. 9,480,416, which is incorporated herein by reference and attached to the Appendix of priority application U.S. 63/279,747. Alternatively, apparatus 10 may lack a position sensor near the end effector 100.

The display 118 is coupled with the processor of console 112 and is operable to render images of patient anatomy. Such images may be based on a set of preoperatively or intraoperatively obtained images (e.g., a CT or MRI scan, 3-D map, etc.). The views of patient anatomy provided through the display 118 may also change dynamically based on signals from the position sensor near the end effector 100.

The processor of the console 112 may also drive the display 118 to superimpose the current location of end effector 100 on the images of the patient's anatomy, such as by superimposing an illuminated dot, a crosshair, a graphical representation of end effector 100, or some other form of visual indication.

The fluid source 142 can include a bag containing saline or some other suitable irrigation fluid. The conduit 140 can include a flexible tube that is further coupled with a pump 144, which is operable to selectively drive fluid from the fluid source 142 to the irrigation tube 15 of the apparatus 10. In some variations, such as including a reference electrode 38D and/or 38P as configured in FIG. 5, the conduit 140, fluid source 142, and pump 144 are omitted entirely.

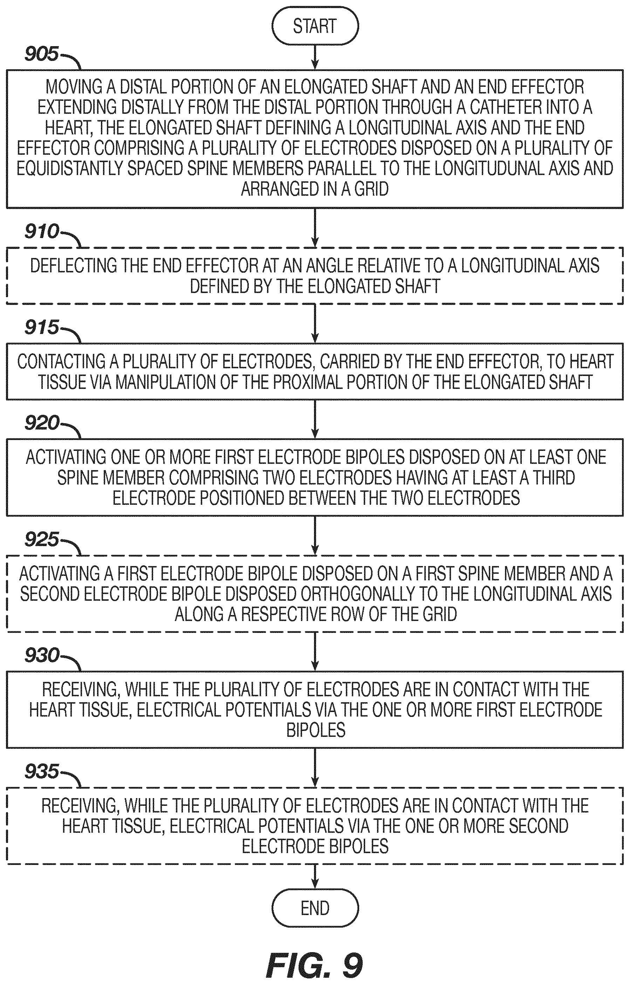

FIG. 9 is a flowchart of an exemplary method of treating a patient utilizing the apparatus according to aspects of the present invention. In step 905, the method may include moving a distal portion of an elongated shaft (e.g., elongated shaft of FIG. 1) and an end effector (e.g., end effector 100) extending distally from the distal portion through a catheter (e.g., catheter 10) and into a heart. The elongated shaft may define a longitudinal axis (e.g., longitudinal axis L-L) and the end effector may include a plurality of electrodes (e.g., electrodes 37) that are disposed on a plurality of equidistantly spaced spine members parallel to the longitudinal axis (e.g., spines 1A, 1B, 2A, 2B, 3A, 3B). The plurality of electrodes may be arranged in a grid (e.g., as shown in FIG. 5) with the plurality of electrodes forming rows of the grid.

In step 910, the method may include moving the end effector from a distal end of the catheter via manipulation of a proximal portion of the elongated shaft (e.g., control handle 16, as shown in FIG. 1). In step 915, the method may include contacting a plurality of electrodes (e.g., electrodes 37), carried by the end effector (e.g., end effector 100), to heart tissue via manipulation of the proximal portion of the elongated shaft. In step 920, the method may include activating one or more first electrode bipoles (e.g., electrode bipole 37A) disposed on at least one spine member. The first electrode bipole may consist of two electrodes having at least a third electrode positioned between the two electrodes. As discussed with respect to FIG. 5, a bipole configuration that skips every other electrode on a given spine allows for the shortened end effector 100 to provide a longitudinal gap distance Lg1 approximately equivalent to the lateral gap distance Lg2 of the shortened end effector 100.

In optional step 925, the method may include activating a first electrode bipole disposed on a first spine member and a second electrode bipole (e.g., lateral bipole 37B) disposed orthogonally to the longitudinal axis along a respective row of the grid.

In step 930, the method may include receiving, while the plurality of electrodes are in contact with the heart tissue, electrical potentials via the one or more first electrode bipoles (e.g., longitudinal bipoles 37A). In optional step 935 the method may include receiving, while the plurality of electrodes are in contact with the heart tissue, electrical potentials via the one or more second electrode bipoles (e.g., lateral bipoles 37B).

The descriptions contained herein are examples of embodiments of the invention and are not intended in any way to limit the scope of the invention. As described herein, the invention contemplates many variations and modifications of system components, including alternative combinations of components illustrated in separate figures, alternative materials, alternative component geometries, and alternative component placement. Modifications and variations apparent to those having skilled in the pertinent art according to the teachings of this disclosure are intended to be within the scope of the claims which follow.

### EMBODIMENTS

**The following is a list of embodiments that may or may not be claimed hereinafter:**
1. An apparatus comprising:
   a tubular member comprising a proximal portion and a distal portion defining a longitudinal axis, the tubular member configured to be manipulated at the proximal portion to position the distal portion into a heart of a patient, the distal portion having a cross-section disposed about the longitudinal axis, the cross-section intersecting first and second orthogonal planes that extend along the longitudinal axis, the cross section of the distal portion includes a first opening intersecting the first orthogonal plane and the second orthogonal plane and extending along the longitudinal axis;
   six apertures disposed circumferentially about the first opening, each of the apertures configured to receive a spine; and
   an end effector coupled to the distal portion, comprising first, second, and third loop members, each of the loop members including two spines and a connector that connects the two spines, the first, second, and third loop members being configured such that each connector of each of the first, second, and third loop members overlap at a distal vertex, and the end effector being configured to be delivered through vasculature in a collapsed configuration and expand in the heart to a deployed configuration such that in the deployed configuration, each of the loop members defines a plane.
2. The apparatus of embodiment 1, the two spines of the first loop member attached to non- adjacent apertures of the cross-section of the distal portion, the two spines of the second loop member attached to immediately adjacent apertures, and the two spines of the third loop member attached to non-adj acent apertures of the cross-section such that the first loop member and the third loop member do not intersect the second loop member.
3. The apparatus of embodiment 1, the first loop member and the third loop member comprising outer loops and the second loop member comprising an inner loop such that the outer loops intersect one another along the longitudinal axis and do not intersect the inner loop along the longitudinal axis.
4. The apparatus of embodiment 1, in which four apertures are disposed on one side of the second orthogonal plane with three apertures disposed on one side of the first orthogonal plane and three apertures disposed on the other side of the first orthogonal plane.
5. The apparatus of embodiment 1, in which the second loop member is on one side of the second orthogonal plane.
6. The apparatus of embodiment 1, the two spines of each loop member extending along the longitudinal axis, each of the two spines comprising a plurality of electrodes comprising at least a first electrode, a second electrode, and a third electrode disposed thereon,
   each electrode of the plurality of electrodes being spaced from an immediately adjacent electrode on a respective spine of the two spines by a first gap length,
   the first electrode and third electrode of a respective spine of the two spines forming a first bipole,
   each respective electrode of a first spine being spaced from a corresponding electrode of a second spine by a second gap length that measures approximately twice the first gap length, and
   each electrode of the first spine forming second bipoles with the corresponding electrode of the second spine.
7. The apparatus of embodiment 6, each spine being configured such that in the deployed configuration each spine of the two spines of the first, second, and third loop members are approximately parallel to each other such that the plurality of electrodes are positioned to define an electrode grid.
8. The apparatus of embodiment 6, each respective electrode of the first spine being linearly spaced along the first spine and forming respective overlapping electrode pairs.
9. The apparatus of embodiment 6, the first gap length between immediately adjacent electrodes measured from a center of each of the immediately adjacent electrodes, optionally each spine
   comprising a plurality of spine segments each having a length equal to the first gap length less a length of a respective electrode, each spine member comprising eight electrodes, further optionally each of the eight electrodes having an electrode length measuring from approximately 100 microns to approximately 750 microns.
10. The apparatus of embodiment 6, the first gap length comprises a length selected from approximately 1 millimeter, approximately 1.2 millimeters, and approximately 2.4 millimeters.
11. The apparatus of embodiment 6, the plurality of electrodes being configured to receive electrical potentials from the heart when the end effector is in the deployed configuration and/or to ablate heart tissue when the end effector is in the deployed configuration.
12. An end effector having a distal end and a proximal end, the distal end configured to be delivered through vasculature in a collapsed configuration and expand in the heart to a deployed configuration, the end effector comprising a plurality of electrodes disposed on a plurality of equidistantly spaced spine members parallel to a longitudinal axis, the plurality of electrodes arranged in a grid,
   the plurality of equidistantly spaced spine members forming columns of the grid such that within each respective column the plurality of electrodes are spaced from an immediately adjacent electrode by a first gap length,
   the plurality of electrodes forming rows of the grid across the plurality of spine members, such that within each respective row the plurality of electrodes are spaced from an immediately adjacent electrode by a second gap length that measures approximately twice the first gap length.
13. The end effector of embodiment 12, the plurality of spine members being configured such that in the deployed configuration the plurality of spine members are approximately parallel to each other and approximately planar such that the plurality of electrodes are positioned in the grid to define an electrode array.
14. The end effector of embodiment 12, being configured such that the plurality of electrodes comprise a first electrode, a second electrode, a third electrode, on each spine member of the plurality of spine members,
   the first electrode of a first spine member and the third electrode of the first spine member forming a first bipole,
   each electrode of the first spine member forming a plurality of second bipoles with a corresponding electrode of a second spine member positioned immediately laterally adjacent to the first spine member.
15. The end effector of embodiment 12, each electrode of the plurality of electrodes having (i) an electrode length measuring from approximately 100 microns to approximately 750 microns, or (ii) an electrode length measuring approximately 500 microns.
16. The end effector of embodiment 12, the first gap length measuring a length selected from approximately 1 millimeter, approximately 1.2 millimeters, and approximately 2.4 millimeters.

## Claims

1. An end effector having a distal end and a proximal end, the distal end configured to be delivered through vasculature in a collapsed configuration and expand in the heart to a deployed configuration, the end effector comprising a plurality of electrodes disposed on a plurality of equidistantly spaced spine members parallel to a longitudinal axis, the plurality of electrodes arranged in a grid,
the plurality of equidistantly spaced spine members forming columns of the grid such that within each respective column the plurality of electrodes are spaced from an immediately adjacent electrode by a first gap length,
the plurality of electrodes forming rows of the grid across the plurality of spine members, such that within each respective row the plurality of electrodes are spaced from an immediately adjacent electrode by a second gap length that measures approximately twice the first gap length.

2. The end effector of claim 1, the plurality of spine members being configured such that in the deployed configuration the plurality of spine members are approximately parallel to each other and approximately planar such that the plurality of electrodes are positioned in the grid to define an electrode array.

3. The end effector of claim 1 or claim 2, being configured such that the plurality of electrodes comprise a first electrode, a second electrode, a third electrode, on each spine member of the plurality of spine members,
the first electrode of a first spine member and the third electrode of the first spine member forming a first bipole,
each electrode of the first spine member forming a plurality of second bipoles with a corresponding electrode of a second spine member positioned immediately laterally adjacent to the first spine member.

4. The end effector of any preceding claim, each electrode of the plurality of electrodes having (i) an electrode length measuring from approximately 100 microns to approximately 750 microns, or (ii) an electrode length measuring approximately 500 microns.

5. The end effector of any preceding claim, the first gap length measuring a length selected from approximately 1 millimeter, approximately 1.2 millimeters, and approximately 2.4 millimeters.

6. The end effector of any preceding claim, the plurality of electrodes being configured to receive electrical potentials from the heart when the end effector is in the deployed configuration.

7. The end effector of any preceding claim, the plurality of electrodes being configured to ablate heart tissue when the end effector is in the deployed configuration.

8. The end effector of any preceding claim, the first gap length between immediately adjacent electrodes measured from a center of each of the immediately adjacent electrodes.

9. The end effector of any preceding claim, each spine member comprising a plurality of spine segments each having a length equal to the first gap length less a length of a respective electrode.

10. The end effector of any preceding claim, wherein each spine member comprises eight electrodes.

11. An apparatus comprising:
the end effector of any preceding claim, the end effector further comprising first, second, and third loop members, each of the loop members including two spine members of the plurality of spine members and a connector that connects the two spine members, the first, second, and third loop members being configured such that each connector of each of the first,
second, and third loop members overlap at a distal vertex, and wherein in the deployed configuration, each of the loop members defines a plane;
a tubular member comprising a proximal portion and a distal portion defining a longitudinal axis, the tubular member configured to be manipulated at the proximal portion to position the distal portion into a heart of a patient, the distal portion having a cross-section disposed about the longitudinal axis, the cross-section intersecting first and second orthogonal planes that extend along the longitudinal axis, the cross section of the distal portion includes a first opening intersecting the first orthogonal plane and the second orthogonal plane and extending along the longitudinal axis; and
six apertures disposed circumferentially about the first opening, each of the apertures configured to receive a spine member.

12. The apparatus of claim 11, the two spine members of the first loop member attached to non-adjacent apertures of the cross-section of the distal portion, the two spine members of the second loop member attached to immediately adjacent apertures, and the two spine members of the third loop member attached to non-adjacent apertures of the cross-section such that the first loop member and the third loop member do not intersect the second loop member.

13. The apparatus of claim 11 or claim 12, the first loop member and the third loop member comprising outer loops and the second loop member comprising an inner loop such that the outer loops intersect one another along the longitudinal axis and do not intersect the inner loop along the longitudinal axis.

14. The apparatus of any one of claims 11, 12 or 13, in which four apertures are disposed on one side of the second orthogonal plane with three apertures disposed on one side of the first orthogonal plane and three apertures disposed on the other side of the first orthogonal plane.

15. The apparatus of any one of claims 11 to 14, in which the second loop member is on one side of the second orthogonal plane.
